# EUROPEAN PATENT APPLICATION

(11) **EP 4 501 315 A1**
(43) Date of publication of application: **05.02.2025**
(21) Application number: 23189629.1
(22) Date of filing: 04.08.2023
(51) Int. Cl.: A61K 9/10, A61K 31/519, A61K 47/12, A61K 47/44

(54) **LIQUID FORMULATIONS OF FOLATES**

(71) Applicant: Lesaffre et Compagnie, 75001 Paris (FR)
(72) Inventor: GUARNERIO, Diego, 20832 DESIO (MB) (IT); GIOVANNONE, Daniele, 20832 DESIO (MB) (IT); BIANCHI, Davide, 20832 DESIO (MB) (IT)
(74) Representative: Bianchetti & Minoja with Trevisan & Cuonzo IPS SRL

(57) **Abstract**

The invention relates to formulation of folates, in particular pf D-glucosamine 5-methyl-(6S)-tetrahydrofolate, in form of suspension in a mixture of medium chain triglyceride oil and stearin. Medium chain triglyceride oil and stearin having a water content lower than 0.5 % and a content of free glycerols and/or ethylene glycol lower than 2% by weight are preferably used. The invention also discloses a process for the preparation of said formulations.

## Description

The present invention refers to folate formulations in form of suspension in a mixture of medium chain triglyceride oil and stearin.

### Background of the Invention

Folic acid, i.e. N-[4-[[(2-amino-1,4-dihydro-4-oxo-6-pteridinyl)methyl]amino]benzoyl]-L-glutamic acid, and folate, the anion thereof, are forms of the water-soluble vitamin B9 and the precursors of dihydrofolic and tetrahydrofolic acids, and of the respective anions thereof. They occur naturally in food, mostly as conjugates thereof, particularly in liver, kidneys, yeast, fruit and leafy green vegetables, and can also be taken as supplements. Commercially available folic acid, as the above mentioned derivatives thereof, are yet prepared synthetically.

Folic acid occurs as a yellow or yellowish-orange crystalline powder and is very slightly soluble in water and insoluble in alcohol; it is readily soluble in dilute solutions of alkali hydroxides and carbonates. Aqueous solutions of folic acid are heat sensitive and rapidly decompose in the presence of light and/or riboflavin; solutions should be therefore stored in a cool place, protected from light.

Vitamins of the B-complex group help the body to convert carbohydrates into glucose, which is metabolized to produce energy. These vitamins are essential in the breakdown of fats and proteins and play an important role in maintaining muscle tone in the digestive tract and in promoting health of the nervous system, skin, hair, eyes, mouth and liver.

It is known that folic acid is necessary for the production and maintenance of new cells. This is especially important during periods of rapid cell division and growth such as infancy and pregnancy. Folate is needed to replicate DNA. Thus folate deficiency hinders DNA synthesis and cell division, affecting most clinically the bone marrow, a site of rapid cell turnover. Because RNA and protein synthesis are not hindered, large red blood cells, i.e. megaloblasts, are produced, resulting in macrocytic anemia, such as megaloblastic anemia (as may be seen in celiac disease) and in anemias of nutritional origin, or in pregnancy, infancy, or childhood. Accordingly, both adults and children need folate to make normal red blood cells and prevent anemia. Folate also helps prevent changes to DNA that may lead to cancer.

Tetrahydrofolic acid and derivatives thereof are highly unstable, particularly due to their susceptibility to oxidation.

In particular, 5-methyltetrahydrofolic acid has importance as a drug ingredient mainly in oncology, as concomitant therapy with methotrexate and 5-fluorouracil treatment, and in the treatment of folic acid deficiency anaemia associated with pregnancy, antibiotic therapy etc.

FR 2137186 describes water soluble alkanol ammonium salts of folic acids.

Among folates and reduced folates, the calcium salts can be mentioned as the most relatively stable derivatives: US 5817659 and US 6441168 disclose crystalline salts, preferably calcium salts, of 5-methyl-(6R,S)-, -(6S)- or - (6R)-tetrahydrofolic acid having a water of crystallization of at least one equivalent per equivalent of said acid. Calcium 5-methyltetrahydrofolate is the only folic acid derivative on the market which is able directly to penetrate the blood/brain barrier without further metabolism. Naturally occurring 5-methyltetrahydrofolic acid is solely in the S form; the R form is biochemically inactive and is excreted through the kidney.

The insolubility in water of these salts has been reported. Besides, a number of compositions for human and animal consumption, comprising either folates and/or reduced folates, are disclosed, in various forms and together with vitamins, arginine, lysine, thiamine and/or other active ingredients, for instance in US 5817659, US 5997915, US 6093703, US 6241996, US 6254904, US 6261600, US 6271374, US 6440450 , US 6441168, US 6444218, US 6451360, US 6514973, US 6544944, US 6596721, US 6605646, US 6673381, US 6808725, US 6914073, US 6921754, US 6995158, US 20020094970, US 20040219262, US20050113332, US 20060063768, either as a nutritional supplement or for the treatment and prevention of various diseases such as, for instance, neurological, pathopsychological, cardiovascular diseases, arthritic and inflammation conditions.

EP 2 245 032 discloses water soluble crystalline or amorphous D-glucosamine and D-galactosamine folate salts, in particular the D-glucosamine salt of 5-methyl-5-methyl-(6 S)-tetrahydrofolate.

Liquid formulations of folates are commonly available as dietary supplements or prescription medications.

Liquid formulations of folates offer several advantages over traditional tablet or capsule forms, particularly for individuals who may have difficulty swallowing or absorbing solid oral medications. Liquid folate supplements are generally easier to consume, especially for children, older adults, or individuals with swallowing difficulties.

Liquid folate formulations typically come in the form of oral solutions or suspensions. These products contain a measured amount of folate, usually expressed in micrograms (mcg) or milligrams (mg). They are designed to be taken orally by measuring the appropriate dose using a dropper, syringe, or provided measuring device.

In view of the solubility characteristics of folates, most known liquid formulations are essentially aqueous. The known liquid formulations are however affected by stability problems limiting their widespread use.

### Description of the invention

It has now been found that stable oily liquid formulations of folates may be obtained by suspending the active ingredient in a mixture of medium chain triglyceride oil and stearin.

In a first embodiment, the present invention accordingly provides formulations of folates in form of suspension in a mixture of medium chain triglyceride oil and stearin.

The term "folate " refers to folic acid, dihydrofolic, tetrahydrofolic, unsubstituted or substituted with a 5-methyl-, 5-formyl-, 10-formyl-, 5,10-methylene-, 5,10-methenyl-moiety, in a (6R,S), (6S) or a (6R) configuration, salts or derivatives thereof.

Sodium, calcium or glucosamine salts, in amorphous or crystalline form, are preferred, in particular the D-glucosamine salts disclosed in EP 2 245 032.

Examples of preferred folates include D-glucosamine-folate, D-galactosamine-folate, D-glucosamine (6R,S)-tetrahydrofolate, D-glucosamine (6S)-tetrahydrofolate, D-glucosamine (6R)-tetrahydrofolate; D-galactosamine (6R, S)-tetrahydrofolate, D-galactosamine (6S)-tetrahydrofolate, D-galactosamine (6R)-tetrahydrofolate; D-glucosamine 5-methyl-(6R,S)-tetrahydrofolate, D-glucosamine 5-methyl-(6S)-tetrahydrofolate, D-glucosamine 5-methyl-(6R)-tetrahydrofolate; D-galactosamine 5-methyl-(6R,S)-tetrahydrofolate, D-galactosamine 5-methyl-(6S)-tetrahydrofolate, D-galactosamine 5-methyl-(6R)-tetrahydrofolate. D-glucosamine 5-methyl-(6S)-tetrahydrofolate is particularly preferred.

The term "medium chain triglyceride oil" refers to triglycerides with two or three fatty acids having an aliphatic chain of 6-12 carbon atoms, i.e. medium-chain fatty acids. Said oils are commercially available from several sources and may be obtained from palm kernel oil and coconut oil.

The weight ratio of medium chain triglyceride oil to stearin is preferably comprised from 10: 1 to 90: 1.
Stearin (E 570) and MCT (medium chain triglyceride) oils have preferably a water content lower than 0.5% by weight.

The concentration by weight of folate in the formulations of the invention ranges from 0.01% to 5.0%

The formulations of the invention may also comprise other excipients such as emulsifiers, oils, gelling agents. Examples of said excipients include glycerol derivatives, mono-diglycerides of fatty acids, beeswax, polysorbate 80 and polysorbate 20, lactic esters of mono-diglycerides of fatty acids, sodium carboxymethylcellulose, olive oil, sunflower oil.

The water content of the excipients is preferably less than 0.5% by weight.

Preferably, each of the excipients reported in the previous points has a content of free glycerols and/or ethylene glycol, lower than 2 % by weight.

According to a second embodiment, the invention provides a process for the preparation of stable oily suspension of folates, comprising:
- Dispersing stearin in medium chain triglyceride (MCT) oil at 70°C
- Cooling under stirring to 30°C;
- Adding the folate to the cloudy oily suspension under stirring at room temperature.

The formulations of the invention are characterized by stability of the active ingredients at temperatures up to 40°C for 90 days or longer at relative humidity of 75% (RH), without any noteworthy color changes.

Formulations prepared using conventional excipients and carriers, such as monoglyceryl dibehenate, polyoxyethylene (20) sorbital, beeswax derivative, silicon dioxide, polyglyceryl 3 oleate, magnesium stearate, sunflower lecithin, glyceril distearate, polyethylene glycol (PEG 400) did not prove successful in securing satisfactory stability: a rapid browning of the suspension prepared with said components was observed, with consequent degradation of the active ingredient. Even the addition of antioxidant agents (such as: ascorbic acid, α-tocopherol, sodium sulfite, butylated hydroxyanisole, sodium ascorbate) had no positive effect on the stability of the suspension.

The invention is described in more detail in the following examples.

### Example 1

| **COMPONENTS** | **AMOUNT PER DROPPER** (DOSE: 400 µG/ML) | |
|---|---|---|
| **Active Ingredient** | | |
| 5-MTHF amorphous glucosamine salt | 0.10 % | (*) 27,00 mg |

| **Excipients** | | |
|---|---|---|
| Medium Chain Triglycerides (MCT oil) | 94.90 % | 26478,00 mg |
| Stearin | 5 % | 1395.00 mg |
| | | |
| **TOTAL WEIGHT:** | **100 %** | **27900.00 mg** |

| | | |
|---|---|---|
| (*) Equivalent to **460 mcg of 5-MTHF acid** (calculated on drug substance assay of 51.10%) and including **15 % overdosage.** | | |

### Example 2

| **COMPONENTS** | **AMOUNT PER DROPPER** (DOSE: 3.5 MG/ML) | |
|---|---|---|
| **Active Ingredient** | | |
| 5-MTHF amorphous glucosamine salt | 0.85 % | (*) 236.40 mg |

| **Excipients** | | |
|---|---|---|
| Medium Chain Triglycerides (MCT oil) | 94.15 % | 26268,60 mg |
| Stearin | 5 % | 1395.00 mg |
| | | |
| **TOTAL WEIGHT:** | **100 %** | **27900.00 mg** |

| | | |
|---|---|---|
| **(*)** Equivalent to **4.0 mg of 5-MTHF acid** (calculated on drug substance assay of 51.10%) and including **15 % overdosage.** | | |

### Example 3

| **COMPONENTS** | **AMOUNT PER DROPPER** (DOSE: 400 µG/ML) | |
|---|---|---|
| **Active Ingredient** | | |
| 5-MTHF amorphous calcium salt | 0.06 % | (*) 16.80 mg |

| **Excipients** | | |
|---|---|---|
| Medium Chain Triglycerides (MCT oil) | 94.94 % | 26488,20 mg |
| Stearin | 5 % | 1395.00 mg |
| | | |
| **TOTAL WEIGHT:** | **100 %** | **27900.00 mg** |

| | | |
|---|---|---|
| **(*)** Equivalent to **460 mcg of 5-MTHF acid** (calculated on drug substance assay of 82.00%) and including **15 % overdosage.** | | |

### Example 4

| **COMPONENTS** | **AMOUNT PER DROPPER** (DOSE: 400 µG/ML) | |
|---|---|---|
| **Active Ingredient** | | |
| 5-MTHF crystalline calcium salt (type I) | 0.06 % | (*) 16.59 mg |

| **Excipients** | | |
|---|---|---|
| Medium Chain Triglycerides (MCT oil) | 94.94 % | 26488,41 mg |
| Stearin | 5 % | 1395.00 mg |
| | | |
| **TOTAL WEIGHT:** | **100 %** | **27900.00 mg** |

| | | |
|---|---|---|
| (*) Equivalent to **460 mcg of 5-MTHF acid** (calculated on drug substance assay of 83.10%) and including **15 % overdosage.** | | |

### Example 5

| **COMPONENTS** | **AMOUNT PER DROPPER** (DOSE: 400 µG/ML) | |
|---|---|---|
| **Active Ingredient** | | |
| 5-MTHF crystalline calcium salt (type C) | 0.06 % | (*° 17,40 mg |

| **Excipients** | | |
|---|---|---|
| Medium Chain Triglycerides (MCT oil) | 94.94 % | 26487,60 mg |
| Stearin | 5 % | 1395.00 mg |
| | | |
| **TOTAL WEIGHT:** | **100 %** | **27900.00 mg** |

| | | |
|---|---|---|
| **(*)** Equivalent to **460 mcg of 5-MTHF acid** (calculated on drug substance assay of 79,00%) and including **15 % overdosage.** | | |

### Example 6

| **COMPONENTS** | **AMOUNT PER DROPPER** (DOSE: 400 µG/ML) | |
|---|---|---|
| **Active Ingredient** | | |
| Folic acid | 0.05 % | (*) 13.80 mg |

| **Excipients** | | |
|---|---|---|
| Medium Chain Triglycerides (MCT oil) | 94.95 % | 26491.20 mg |
| Stearin | 5 % | 1395.00 mg |
| | | |
| **TOTAL WEIGHT:** | **100 %** | **27900.00 mg** |

| | | |
|---|---|---|
| **(*)** Equivalent to **460 mcg of 5-MTHF acid** (calculated on drug substance assay of 100.00%) and including **15 % overdosage.** | | |

### Example 7

| **COMPONENTS** | **AMOUNT PER DROPPER** (DOSE: 400 µG/ML) | |
|---|---|---|
| **Active Ingredient** | | |
| 5-MTHF amorphous glucosamine salt | 0.10 % | (*) 27,00 mg |

| **Excipients** | | |
|---|---|---|
| Medium Chain Triglycerides (MCT oil) | 90.90 % | 25362.00 mg |
| Stearin | 3 % | 837,00 mg |
| Mono-diglycerides of fatty acids (E-471) | 4% | 1116.00 mg |
| Polysorbate 80 | 2% | 558,00 mg |
| **TOTAL WEIGHT:** | **100 %** | **27900.00 mg** |

| | | |
|---|---|---|
| (*) Equivalent to **460 mcg of 5-MTHF acid** (calculated on drug substance assay of 51.10%) and including **15 % overdosage.** | | |

### Example 8 Stability tests

The stability of the formulation of example 1 was tested at 5°C, 25 °C, 30 °C and 40 °C at 75 % RH. The normalized results are reported in the following tables:

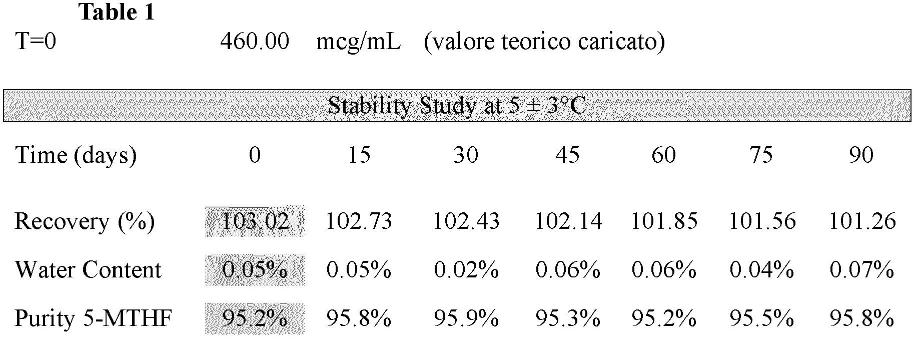

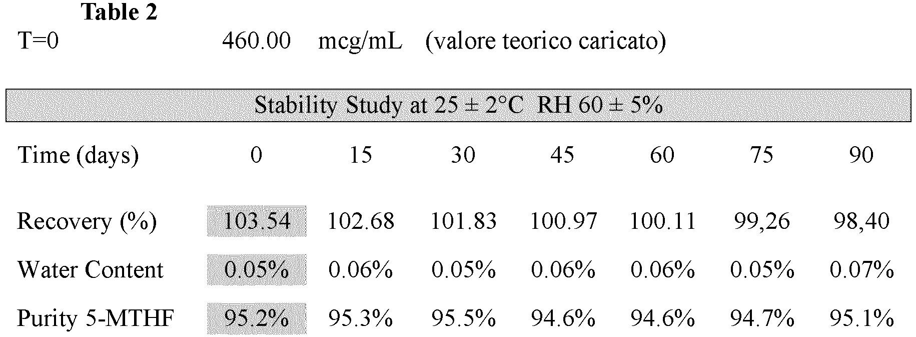

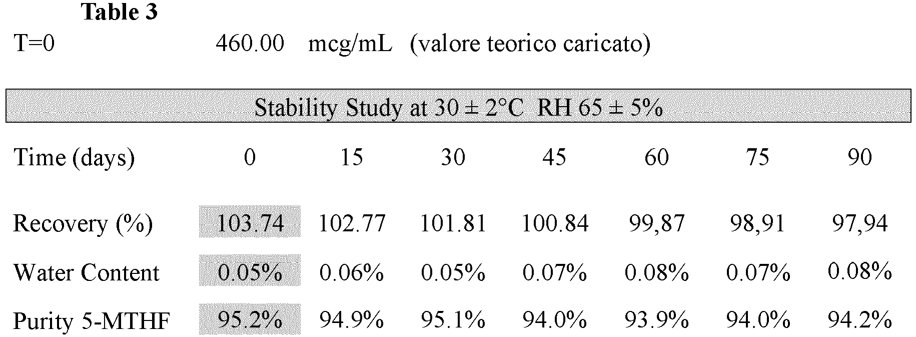

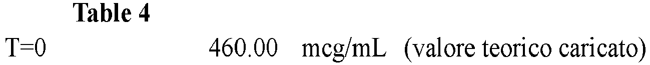

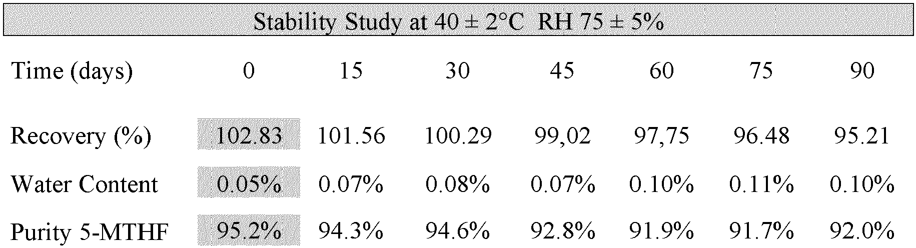

## Claims

1. A formulation of folates in form of suspension in a mixture of medium chain triglyceride oil and stearin.

2. A formulation according to claim 1 wherein the folate are in form of sodium, calcium or glucosamine salts, in amorphous or crystalline form.

3. A formulation according to claim 1 or 2 wherein the folates are selected from folate, -dihydrofolate, -tetrahydrofolate, unsubstituted or substituted with a 5-methyl-, 5-formyl-, 10-formyl-, 5,10-methylene-, 5,10-methenyl-moiety, in a (6R,S), (6S) or a (6R) configuration, preferably D-glucosamine-folate, D-galactosamine-folate, D-glucosamine (6R,S)-tetrahydrofolate, D-glucosamine (6S)-tetrahydrofolate, D-glucosamine (6R)-tetrahydrofolate; D-galactosamine (6R,S)-tetrahydrofolate, D-galactosamine (6S)-tetrahydrofolate, D-galactosamine (6R)-tetrahydrofolate; D-glucosamine 5-methyl-(6R,S)-tetrahydrofolate, D-glucosamine 5-methyl-(6S)-tetrahydrofolate, D-glucosamine 5-methyl-(6R)-tetrahydrofolate; D-galactosamine 5-methyl-(6R,S)-tetrahydrofolate, D-galactosamine 5-methyl-(6S)-tetrahydrofolate, D-galactosamine 5-methyl-(6R)-tetrahydrofolate, more preferably D-glucosamine 5-methyl-(6S)-tetrahydrofolate.

4. A formulation according to any one of claims from 1 to 3 wherein medium chain triglyceride oil and stearin have a water content lower than 0.5 % and a content of free glycerols and/or ethylene glycol lower than 2% by weight.

5. A formulation according to any one of claims from 1 to 4 comprising excipients and/or emulsifiers.

6. A formulation according to claim 5 wherein the excipients are selected from the group of glycerol derivates, mono-diglycerides of fatty acids, beeswax, polysorbate 80 polysorbate 20, lactic esters of mono-diglycerides of fatty acids, sodium carboxymethylcellulose, olive oil, sunflower oil. lecithins.

7. A formulation according to claim 6, wherein the excipients have a water content lower than 0.5% by weight.

8. A formulation according to claim 6 or 7 wherein the excipients have a content of free glycerols and/or ethylene glycol lower than 2 % by weight.

9. A formulation according to any one of claims from 1 to 8 wherein the weight ratio of medium chain triglyceride oil to stearin is comprised from 10:1 to 90:1.

10. A formulation according to any one of claims from 1 to 9 wherein the concentration by weight of folic acid, salts or derivatives thereof ranges from 0.01% to 5.0% by weight.

11. A process for the preparation of the formulations of claims 1-10 comprising:
- Dispersing stearin in MCT oil at 70°C;
- Cooling under stirring to 30°C;
- Adding the folate to the cloudy oily suspension under stirring at room temperature.

12. A process according to claim 11 wherein the folate is a glucosamine salt.

13. A process according to claim 12 wherein the folate is D-glucosamine 5-methyl-(6S)-tetrahydrofolate.
